# EUROPEAN PATENT SPECIFICATION

(11) **EP 2 799 870 B1**
(45) Date of publication and mention of the grant of the patent: **24.10.2018**
(21) Application number: 12861523.4
(22) Date of filing: 28.12.2012
(51) Int. Cl.: G01N 33/543, G01N 33/53

(54) **METHOD FOR REDUCING ADSORPTION OF BUBBLES**
VERFAHREN ZUR VERRINGERUNG DER ADSORPTION VON BLASEN
PROCÉDÉ POUR RÉDUIRE L'ADSORPTION DE BULLES

(30) Priority: 28.12.2011 JP 2011289826
(43) Date of publication of application: 05.11.2014
(73) Proprietor: Denka Seiken Co., Ltd., Chuo-ku Tokyo 103-8338 (JP)
(72) Inventor: KANO, Mayumi, Gosen-shi Niigata 959-1695 (JP); MIZUE, Hiromi, Gosen-shi Niigata 959-1695 (JP)
(74) Representative: Blodig, Wolfgang
(86) International application number: PCT/JP2012/084121
(87) International publication number: WO 2013/100137

(56) References cited:
- EP-A1- 2 325 639
- EP-A2- 1 813 931
- WO-A1-2009/120363
- DE-A1- 4 309 529
- GB-A- 1 503 547
- JP-A- 2006 284 231
- JP-A- 2007 205 732
- JP-A- 2009 145 357
- JP-A- 2009 300 454
- JP-A- 2010 266 462

## Description

### [Technical Field]

The present invention relates to a method for reducing adsorption of bubbles onto a cell side surface in an immunoassay using a dry plastic cell.

### [Background Art]

An immunological analysis method is widely used in clinical examinations of serum, plasma, urine, feces, cerebrospinal fluid, and the like. Recently, automatic analyzers capable of automatically carrying out a series of analysis from reaction to measurement have been generally used because they enable simple and rapid measurement.

The automatic analyzers are divided into two classes: analyzers whose cell into which a reaction liquid is placed is washed and repeatedly used, and analyzers whose cell is disposable. Disposable cells are a dry plastic cell in many cases.

In automatic analyzers using a dry plastic cell, when a reagent for automatic analyzers using other wet cells is used, analysis performance may be deteriorated in several cases. However, causes thereof have not been elucidated, and the problems are not solved.

On the other hand, adding a surfactant to an immunological analysis reagent is known, and has been often used as a means for controlling reactivity such as avoiding influence of a matrix or a means for amplifying an amount of change in absorbance (see Patent Documents 1 and 2).

### [Citation List]

### [Patent Document]

[Patent Document 1] JP-A-2005-241415
[Patent Document 2] JP-A-2006-126166
WO2009/120363 A1 discloses an aqueous solution containing a buffer and an organosilicone surfactant for applying to a channel.
DE 4309529 A1 discloses quantitative or qualitative analyte determination by photometric analysis.
EP 1813931 A1 discloses a sheath liquid for a particle analyzer.

### [Summary of the Invention]

### [Problems to be Solved by the Invention]

The present invention relates to providing a means for improving measurement accuracy in an immunoassay using a dry plastic cell.

### [Means for Solving the Problems]

The inventors of the present application have intensively studied, and they have found that, in an immunoassay using a dry plastic cell, bubbles are often adsorbed on a side surface of the cell which is a surface to be lighted (a spectrophotometry surface), thus deteriorating measurement accuracy. Then, they have found that the adsorption of the bubbles can be easily reduced by allowing a surfactant to be present in a reaction and measurement system.

Accordingly, the present invention relates to the following 1) to 5).
1) A method for reducing adsorption of bubbles onto a cell side surface in an immunoassay, the method comprising carrying out a reaction and/or measurement in a presence of a surfactant, wherein the immunoassay comprises carrying out an antigen-antibody reaction in a dry plastic cell using an immunoassay reagent which immunologically reacts with a substance to be measured in a sample and carrying out an optical measurement of a resultant reacted product in the presence of a surfactant, wherein the surfactant is a poly(oxyethylene) sorbitan fatty acid ester or a polyoxyethylene tribenzyl phenyl etehr, wherein a concentration of the surfactant in the system of the reaction and/or measurement is 0.01 to 0.3%, and wherein the immunoassay is an immunoagglutination assay.
2) The method according to the above 1), wherein the immunoagglutination assay is a latex agglutination assay.
3) Use of an immunoassay reagent comprising a surfactant for reducing adsorption of bubbles onto a cell side surface in an immunoassay, wherein the immunoassay comprises carrying out an antigen-antibody reaction in a dry plastic cell using an immunoassay reagent which immunologically reacts with a substance to be measured in a sample and carrying out an optical measurement of a resultant reacted product in the presence of the surfactant, wherein the surfactant is a poly(oxyethylene)sorbitan fatty acid ester or a polyoxyethylene tribenzyl phenyl ether, wherein a concentration of the surfactant in the system of the reaction and/or measurement is 0.01 to 0.3%, and wherein the immunoassay is an immunoagglutination assay.
4) Use of an immunoassay reagent according to the above 3), wherein the reagent further comprises an immunoagglutination reagent.
5) Use of an immunoassay reagent according to the above 4), wherein the immunoagglutination reagent is a latex agglutination reagent.

### [Effects of the Invention]

According to the method of the present invention, a simple means of allowing a surfactant to be present in a reaction and measurement system makes it possible to effectively reduce adsorption of bubbles onto a spectrophotometry surface of a dry plastic cell, resulting that a measurement value is stabilized and the measurement accuracy is improved in an immunoassay by an automatic analyzer using the cell. Thus, a difference in performance depending upon types of automatic analyzers is reduced, and range of applicable automatic analyzers is expanded.

### [Modes for Carrying out the Invention]

Hereinafter, the method of the present invention is described. Note here that "%" in the present specification denotes a mass basis (w/v%) unless otherwise specified.

The method for reducing adsorption of bubbles onto a cell side surface in an immunoassay according to the present invention comprises carrying out a reaction and/or measurement in a presence of a surfactant, wherein the immunoassay comprises carrying out an antigen-antibody reaction in a dry plastic cell using an immunoassay reagent which immunologically reacts with a substance to be measured in a sample and carrying out an optical measurement of a resultant reacted product.

The immunoassay to which the method of the present invention is applied refers to an analysis method comprising carrying out an antigen-antibody reaction in a dry plastic cell using an immunoassay reagent which immunologically reacts with a substance to be measured in a sample and carrying out an optical measurement of a resultant reacted product.

The immunoassay is an immunoagglutination assay, and a latex agglutination assay using latex particles as insoluble carrier particles is preferable. The immunoagglutination assay is well known as a method for optically detecting agglutination of sensitized particles which are sensitized with an antigen or antibody, and the detection preferably uses a turbidimetric method or a colorimetric method. For example, light in a visible region to a near infrared region, e.g., light with, usually 300 to 1000 nm, preferably 500 to 900 nm, is irradiated from the outside of a cell to detect change in the absorbance or change in intensity of the scattered light, whereby the degree of the agglutination of the sensitized particles is measured.

When the immunoassay is carried out by the immunoagglutination assay, the insoluble carrier particles to be used are not particularly limited, and they may be well-known particles which have been conventionally used in the immunoassay reagent. Examples of the insoluble carrier particles include latex particles such as polyethylene and polystyrene, alumina particles, silica particles, gold colloid, and magnetic particles. Among these insoluble carriers, latex particles, particularly, polystyrene latex particles are suitably used. The size of the latex particle is not particularly limited, but the particle diameter is preferably 30 to 600 nm.

A case where a substance to be measured is an antigen in an immunoagglutination assay is described as one example below.

On the above-mentioned insoluble carrier particles, an antibody which immunologically reacts with an antigen to be measured or an antigen-binding fragment thereof is immobilized. The method of immobilization is also well-known, and it is carried out by a well-known method such as one utilizing physical adsorption or covalent bond. When a suspension of the obtained sensitized particles and a test sample are mixed with each other, the sensitized particles are agglutinated by a substance to be measured (antigen) contained in the test sample, and the absorbance of the sensitized particle suspension is changed. The amount of the change (end-point method) or the rate of the change (rate method) in absorbance is measured. A plurality of standard samples containing the antigen to be measured at various known concentrations are prepared, and they are measured for the amount of change or the rate of change in the absorbance by the above-mentioned method. The concentration of the antigen to be measured in the standard sample is plotted on the abscissa, and the amount of change or the rate of change in the absorbance measured is plotted on the ordinate so as to draw a calibration curve. As to an unknown test sample, the amount of change or the rate of change in the absorbance is measured by the same method, and the measurement results thereof are assigned to the above-mentioned calibration curve. Thus, the antigen in the test sample can be quantified.

Automatic devices capable of carrying out such an immunoagglutination assay are commercially available, and the immunoagglutination assay can be carried out easily and simply using the commercially available automatic devices for immunoagglutination assay.

Examples of the substance to be measured by the immunoassay in the present invention include, as antigens, protein markers such as CRP (C-reactive protein), prostate-specified antigen, ferritin, β-2 microglobulin, myoglobin, hemoglobin, albumin, and creatinine; immunoglobulins such as IgG, IgA, and IgM; various tumor markers; lipoprotein such as LDL, HDL, and TG; antigens of viruses such as influenza virus type A, influenza virus type B, RS virus (RSV), Rhinovirus, rotavirus, norovirus, adenovirus, astrovirus, HAV, HBs, HCV, HIV, and EBV; antigens of bacteria such as Chlamydia trachomatis, hemolytic streptococcus, Bordetella pertussis, Helicobacter pylori, Leptospira, Treponema pallidum, Toxoplasma gondii, Borrelia, Legionella bacteria, Bacillus anthracis, and MRSA; toxin produced by bacteria; a Mycoplasma lipid antigen; peptide hormones such as human chorionic gonadotropin; steroids such as steroid hormones; physiologically active amines such as epinephrine and morphine; vitamins such as vitamin Bs; prostaglandins; antibiotics such as tetracycline; agricultural chemicals; and environmental hormones, but the substance to be measured is not limited thereto. Preferable examples include antigens such as CRP, prostate-specific antigen, ferritin, β-2 microglobulin, and hemoglobin.

When the substance to be measured is an antibody, examples include antibodies which specifically react with antigens including the above-mentioned protein markers, various tumor markers, lipoproteins, viral antigens, bacterial antigens, toxins produced by bacteria, peptide hormones, steroids, physiologically active amines, vitamins, antibiotics, agricultural chemicals, and environmental hormones.

The sample to be used in the immunoasay is not particularly limited as long as the sample contains a substance to be measured, and examples thereof include body fluid such as blood, serum, plasma, urine, feces, saliva, interstitial fluid, cerebrospinal fluid, and swab, or dilutions thereof. Preferable examples include blood, serum, plasma, urine, feces, and cerebrospinal fluid or dilutions thereof.

The dry plastic cell to be used in the method of the present invention means a disposable type cell which is made of material such as polyethylene, polypropylene, a copolymer of polyethylene and polypropylene, and polymethyl methacrylate, and which is in a dry state when it is used.

As the surfactant of the present invention a poly(oxyethylene) sorbitan fatty acid ester or a polyoxyethylene tribenzyl phenyl ether is used.

Among them, preferable examples of the poly(oxyethylene)sorbitan fatty acid ester include polyoxyethylene sorbitan monolaurate, polyoxyethylene sorbitan monopalmitate, polyoxyethylene sorbitan monostearate, and polyoxyethylene sorbitan tristearate.

In the method of the present invention, the surfactant may be contained in the reaction and/or measurement system (which is also referred to as a "reaction-measurement system") in any step between the start of antigen-antibody reaction and the completion of detection and quantification of the amount of the antigen-antibody reaction. It is preferable that the surfactant be contained throughout the period from the start of the antigen-antibody reaction to the detection and quantification.

Therefore, it is preferable that the surfactant be added to the reaction system before or concurrently with the start of the antigen-antibody reaction. Specifically, it may be added when a sample is diluted, or it may be added when an antibody or an antigen is mixed with a sample.

Furthermore, the surfactant may be contained, in advance, in various reagents to be used in an immunoassay, and the present invention also discloses an immunoassay reagent containing such a surfactant.

Herein, examples of the various reagents to be used in an immunoassay include a sample diluent, an antibody/antigen diluent, a solid phase antibody/antigen, a sensitized particle suspension, a washing solution, an enzyme solution, a substrate solution, and a test sample standard solution for preparing a calibration curve. Examples of the immunoassay reagent containing a surfactant include a reagent obtained by adding a surfactant to these reagents, for example, a buffer solution for diluting a sample and a reagent and the like containing an antibody or antigen into which a surfactant is incorporated.

When the immunoagglutination assay is employed, for example, an immunoagglutination reagent containing insoluble carrier particles (sensitized particle) on which an antibody or antigen is immobilized (sensitized) may contain the surfactant.

In this case, the concentration of the sensitized particles in the immunoassay reagent is preferably, but not particularly limited to, 0.01 to 0.5%. The amount of antibody and the amount of antigen in the sensitized particle suspension may be the same as in a routine method, and they are not particularly limited. However, when, for example, an antibody-sensitized latex is used, the amount of antibody in the latex suspension is preferably 0.01 to 2.0 mg/mL.

The concentration of the surfactant in the reaction-measurement system is 0.01 to 0.3% from the viewpoint of reducing adsorption of bubbles. Therefore, when the surfactant is contained in the immunoassay reagent in advance, the surfactant may be contained in the immunoassay reagent so that the concentration in the reaction-measurement system falls in the above-mentioned concentration.

The Blank sample to be used in the immunoassay is not particularly limited as long as it cannot contain a substance to be measured, but it is preferably purified water, physiological saline, a buffer solution, a negative sample, or a dilution thereof.

As shown in Examples below, when the surfactant is allowed to be present in the reaction-measurement system, adsorption of bubbles onto a side surface of a cell as a spectrophotometry surface, that is, a surface to be lighted of a dry plastic cell is reduced. Then, as compared with the case where the surfactant is not allowed to be present, the measurement accuracy is significantly improved. Therefore, when the method of the present invention is used, application range of an automatic analyzer using a dry plastic cell can be expanded.

### [Examples]

Hereinafter, the present invention is described more specifically with reference to Examples. However, the present invention is not limited to the following Examples.

### Examples 1 to 6

### (1) Preparation of reagents

Measurement reagents for an immunoagglutination assay were prepared as follows using an antibody against ferritin.
i) Sensitized particles which support 0.045 mg of an anti-ferritin antibody for 1 mL of a suspension of polystyrene latex having an average particle diameter of 300 nm were suspended in a buffer solution (glycine, pH 7.3) at a concentration of 0.056%, to thereby prepare a latex suspension.
ii) Various surfactants were added to a buffer solution (Tris, pH 8.5) to prepare the below-mentioned reagents A to F. As comparative examples, prepared were reagent G in which a surfactant component had not been added and reagent H in which a protein had been added instead of a surfactant component.

**[Table 1]**

| Reagent | Surfactant (concentration) |
|---|---|
| A | Poly(oxyethylene)alkyl ether (0.1%) |
| B | Polyoxyethylene (80) sorbitan monolaurate (0.3%) |
| C | Polyoxyethylene - polyoxypropylene condensate (0.1%) |
| D | Polyoxyethylene tribenzyl phenyl ether (0.2%) |
| E | Olefin-maleic acid copolymer ammonium salt (0.01%) |
| F | Polystyrene sodium sulfonate (0.1%) |
| G | Not included |
| H | Not included |

| | |
|---|---|
| A to D: Nonionic surfactants (A: "EMULGEN 707" (Kao Corporation), B: "Tween 80" (Wako Pure Chemical Industries, Ltd.), C: "Pluronic F68" (ADEKA), and D: "EMULGEN B66" (Kao Corporation) were used, respectively.) E and F: Anionic surfactants (E: "POLYSTER OM" (NOF CORPORATION) and F: "PS-1" (Tosoh Corporation) were used, respectively.) G: Buffer solution alone H: Buffer solution + protein (2.0%, bovine serum albumin) | |

### (2) Measurement by automatic analyzer

Automatic measurement was carried out by an end-point method using Automatic Analyzer VITROS 5600 (manufactured by Ortho Clinical Diagnostics).

Measurement for a control sample (100 ng/mL) was carried out 10 times in total using the above-mentioned buffer solutions of reagents A to H. To 10.0 µL of the sample solution, 100 µL of the respective buffer solutions of reagents A to H was added, and the mixed solution was stirred and mixed together at 37°C. After the mixed solution was allowed to stand for 5 min, 100 µL of the latex suspension prepared above was added, followed by further stirring and mixing together at 37°C. The agglutination reaction was measured for about 5 minutes in terms of the amount of change in the absorbance, and the standard deviation and coefficient variation (CV (%)) were calculated.

In addition, presence or absence of bubbles adsorbed onto the cell side surface was observed. Results are shown in Table 2 below.

**[Table 2]**

| | Examples | | | | | | Comparative Examples | |
|---|---|---|---|---|---|---|---|---|
| | 1 | 2 | 3 | 4 | 5 | 6 | 1 | 2 |
| Reagent | A | B | C | D | E | F | G | H |
| Average value (mAbs) | 55.7 | 31.3 | 40.5 | 40.1 | 40.9 | 43.2 | Not measurable | 44.9 |
| Standard deviation (mAbs) | 2.4 | 1.6 | 2.2 | 1.9 | 2.3 | 3.5 | - | 4.2 |
| CV (%) | 4.3% | 4.9% | 5.5% | 4.6% | 5.7% | 8.1% | - | 9.5% |
| Adsorption of bubbles | Not observe d | Not observed | Not observed | Not observed | Not observed | Not observed | Observed | Observed |

Examples 1, 3, 5 and 6 are Reference Examples.

Table 2 shows that, with the addition of surfactants, adsorption of bubbles onto the cell side surface is reduced, the standard deviation is reduced and accuracy is improved. Furthermore, it is shown that an effect of addition of the above-mentioned reagents A to F can be obtained regardless of types of surfactants.

### Examples 7 to 12

Measurement reagents for an immunoagglutination assay were prepared as follows using an antibody against ferritin.

### (1) Preparation of reagents

i) Sensitized particles which support 0.045 mg of an anti-ferritin antibody for 1 mL of a suspension of polystyrene latex having an average particle diameter of 300 nm were suspended in a buffer solution (glycine, pH 7.3) at a concentration of 0.056%, to thereby prepare a latex suspension.
ii) A surfactant was added to one or both of a buffer solution (Tris, pH 8.5) and the latex suspension prepared in the above-mentioned i) to prepare reagents I to N. As comparative examples, reagent O in which a surfactant component had not been added was prepared.

**[Table 3]**

| Reagent | |
|---|---|
| I | (i) Buffer solution + 0.2% surfactant, (ii) latex suspension |
| J | (i) Buffer solution + 0.1% surfactant, (ii) latex suspension |
| K | (i) Buffer solution, (ii) latex suspension + 0.2% surfactant |
| L | (i) Buffer solution, (ii) latex suspension + 0.1% surfactant |
| M | (i) Buffer solution + 0.1% surfactant, (ii) latex suspension + 0.1% surfactant |
| N | (i) Buffer solution + 0.05% surfactant, (ii) latex suspension + 0.05% surfactant |
| O | (i) Buffer solution, (ii) latex suspension |

| | |
|---|---|
| Surfactant: Polyoxyethylene tribenzyl phenyl ether ("EMULGEN B66" (Kao Corporation)) | |

### (2) Measurement by automatic analyzer

Automatic measurement was carried out by an end-point method by using Automatic Analyzer VITROS 5600 (manufactured by Ortho Clinical Diagnostics).

Measurement for a control sample (100 ng/mL) was carried out 10 times in total using the above-mentioned reagents I to O. To 10.0 µL of the sample solution, 100 µL of the respective buffer solutions of reagents I to O prepared above was added, and then the mixed solution was stirred and mixed together at 37°C. After the mixed solution was allowed to stand for 5 min, 100 µL of the respective latex suspensions of reagents I to O prepared above was added, followed by further stirring and mixing together at 37°C. The agglutination reaction was measured for about 5 minutes in terms of the amount of change in the absorbance, and the standard deviation and coefficient variation (CV (%)) were calculated. In addition, presence or absence of bubbles adsorbed on the cell side surface was observed. Results are shown in Table 4 below.

**[Table 4]**

| | Examples | | | | | | Comparative Example |
|---|---|---|---|---|---|---|---|
| | 7 | 8 | 9 | 10 | 11 | 12 | 3 |
| Reagent | I | J | K | L | M | N | O |
| Average value (mAbs) | 40.1 | 42.8 | 55.2 | 54.3 | 42.7 | 45.6 | Not measurable |
| Standard deviation (mAbs) | 1.9 | 2.8 | 2.8 | 4.6 | 1.6 | 2.1 | - |
| CV (%) | 4.6% | 6.5% | 5.0% | 8.4% | 3.8% | 4.6% | - |
| Adsorption of bubbles | Not observed | Not observed | Not observed | Not observed | Not observed | Not observed | Observed |

From Table 4, it is shown that even when a surfactant is added in any mode, adsorption of bubbles onto the cell side surface is reduced, and measurement accuracy is improved in a concentration-dependent manner. Furthermore, it is shown that when the surfactant is added to both the buffer solution and the latex suspension, the measurement accuracy is further improved.

### Examples 13 to 14

Measurement reagents for an immunoagglutination assay were prepared as follows using antibodies against ferritin and hemoglobin.

### (1) Reagents to be used

i) Sensitized particles which support 0.045 mg of an anti-ferritin antibody or anti-hemoglobin antibody for 1 mL of a suspension of polystyrene latex having an average particle diameter of 300 nm were suspended in a buffer solution (glycine, pH 7.3) at a concentration of 0.056%, to thereby prepare a latex suspension.
ii) A surfactant was added to a buffer solution (Tris, pH 8.5) and the latex suspension prepared in the above-mentioned i) to prepare reagent P (reagent for measurement of ferritin) and reagent Q (reagent for measurement of hemoglobin).

**[Table 5]**

| Reagent | |
|---|---|
| P | (i) Buffer solution + 0.1% surfactant, (ii) latex suspension + 0.1% surfactant |
| Q | (i) Buffer solution + 0.1% surfactant, (ii) latex suspension + 0.1% surfactant |

| | |
|---|---|
| Surfactant: Polyoxyethylene tribenzyl phenyl ether ("EMULGEN B66" (Kao Corporation)) | |

### (2) Measurement by automatic analyzer

Automatic measurement was carried out by an end-point method by using Automatic Analyzer VITROS 5600(manufactured by Ortho Clinical Diagnostics).

Measurement for a control sample (100 ng/mL) was carried out 10 times in total using the above-mentioned reagents P and Q. To 10.0 µL of the sample solution, 100 µL of the buffer solution of reagent P or Q prepared above was added, and the mixed solution was stirred and mixed together at 37°C. After the mixed solution was allowed to stand for 5 min, 100 µL of the latex suspension of reagent P or Q prepared above was added to the mixture, followed by further stirring and mixing together at 37°C. The agglutination reaction was measured for about 5 minutes in terms of the amount of change in the absorbance, and the standard deviation was calculated. In addition, presence or absence of bubbles adsorbed on the cell side surface was observed. Results are shown in Table 6 below.

**[Table 6]**

| | Examples | |
|---|---|---|
| | 13 | 14 |
| Reagent | P | Q |
| Average value (mAbs) | 42.7 | 36.7 |
| Standard deviation (mAbs) | 1.6 | 1.9 |
| CV (%) | 3.8% | 5.2% |
| Adsorption of bubbles | Not observed | Not observed |

From Table 6, it is shown that even when different antibodies are used, the bubble adsorption onto the cell side surface is reduced with the addition of the surfactant, thus resulting that the measurement accuracy is improved.

## Claims

1. A method for reducing adsorption of bubbles onto a cell side surface in an immunoassay, the method comprising carrying out a reaction and/or measurement in a presence of a surfactant, wherein the immunoassay comprises carrying out an antigen-antibody reaction in a dry plastic cell using an immunoassay reagent which immunologically reacts with a substance to be measured in a sample and carrying out an optical measurement of a resultant reacted product in the presence of the surfactant, wherein the surfactant is a poly(oxyethylene)sorbitan fatty acid ester or a polyoxyethylene tribenzyl phenyl ether, wherein the concentration of the surfactant in the system of the reaction and/or measurement is 0.01 to 0.3%, and wherein the immunoassay is an immunoagglutination assay.

2. The method according to claim 1, wherein the immunoagglutination assay is a latex agglutination assay.

3. Use of an immunoassay reagent comprising a surfactant for reducing adsorption of bubbles onto a cell side surface in an immunoassay, wherein the immunoassay comprises carrying out an antigen-antibody reaction in a dry plastic cell using an immunoassay reagent which immunologically reacts with a substance to be measured in a sample and carrying out an optical measurement of a resultant reacted product in the presence of the surfactant, wherein the surfactant is a poly(oxyethylene)sorbitan fatty acid ester or a polyoxyethylene tribenzyl phenyl ether, wherein a concentration of the surfactant in the system of the reaction and/or measurement is 0.01 to 0.3%, and wherein the immunoassay is an immunoagglutination assay.

4. Use of an immunoassay reagent according to claim 3, wherein the reagent further comprises an immunoagglutination reagent.

5. Use of an immunoassay reagent according to claim 4, wherein the immunoagglutination reagent is a latex agglutination reagent.

## Patentansprüche

1. Verfahren zur Verringerung der Adsorption von Blasen auf einer Seitenfläche einer Küvette in einem Immunassay, wobei das Verfahren das Durchführen einer Reaktion und/oder einer Messung in Anwesenheit eines Tensids umfasst, wobei der Immunassay das Durchführen einer Antigen-Antikörper-Reaktion in einer trockenen Plastik-Küvette unter Verwenden eines Reagenz für einen Immunassay umfasst, welches mit einer in einer Probe zu messenden Substanz immunologisch reagiert, und das Durchführen einer optischen Messung des resultierenden umgesetzten Produkts in Anwesenheit des Tensids, wobei das Tensid ein Poly(oxyethylen)sorbitanfettsäureester oder ein Polyoxyethylentribenzylphenylether ist, wobei die Konzentration des Tensids in dem System der Reaktion und/oder der Messung 0,01 bis 0,3 % beträgt und wobei der Immunassay ein Immunagglutinationsassay ist.

2. Verfahren nach Anspruch 1, wobei Immunagglutinationsassay ein Latex-Agglutinationsassay ist.

3. Verwendung eines Reagenz für einen Immunassay, umfassend ein Tensid zur Verringerung der Adsorption von Blasen auf einer Seitenfläche einer Küvette in einem Immunassay, wobei der Immunassay das Durchführen einer Antigen-Antikörper-Reaktion in einer trockenen Plastik-Küvette unter Verwenden eines Reagenz für einen Immunassay umfasst, welches mit einer in einer Probe zu messenden Substanz immunologisch reagiert, und das Durchführen einer optischen Messung des resultierenden umgesetzten Produkts in Anwesenheit des Tensids, wobei das Tensid ein Poly(oxyethylen)sorbitanfettsäureester oder ein Polyoxyethylentribenzylphenylether ist, wobei die Konzentration des Tensids in dem System der Reaktion und/oder der Messung 0,01 bis 0,3 % beträgt und wobei der Immunassay ein Immunagglutinationsassay ist.

4. Verwendung eines Reagenz für einen Immunassay nach Anspruch 3, wobei das Reagenz ferner ein Reagenz für die Immunagglutination umfasst.

5. Verwendung eines Reagenz für einen Immunassay nach Anspruch 4, wobei das Reagenz für die Immunagglutination ein Reagenz für die Latex-Agglutination ist.

## Revendications

1. Procédé pour réduire l'adsorption de bulles sur une surface du côté cellule d'un immunodosage, le procédé comprenant la mise en oeuvre d'une réaction et/ou d'une mesure en présence d'un tensioactif, dans lequel l'immunodosage comprend la mise en oeuvre d'une réaction antigène-anticorps dans une cellule plastique sèche utilisant un réactif d'immunodosage qui réagit immunologiquement avec une substance devant être mesurée dans un échantillon, et la mise en oeuvre d'une mesure optique d'un produit ayant réagi résultant en présence du tensioactif, dans lequel le tensioactif est un ester d'acide gras et de sorbitan polyoxyéthyléné ou un tribenzylphényléther polyoxyéthyléné, dans lequel la concentration du tensioactif dans le système de la réaction et/ou de la mesure est de 0,01 à 0,3 %, et dans lequel l'immunodosage est un dosage d'immunoagglutination.

2. Procédé selon la revendication 1, dans lequel le dosage d'immuno-agglutination est un dosage d'agglutination de latex.

3. Utilisation d'un réactif d'immunodosage comprenant un tensioactif pour réduire l'adsorption de bulles sur une surface du côté cellule dans un immunodosage, dans laquelle l'immunodosage comprend la mise en oeuvre d'une réaction antigène-anticorps dans une cellule plastique sèche utilisant un réactif d'immunodosage qui réagit immunologiquement avec une substance devant être mesurée dans un échantillon, et la mise en oeuvre d'une mesure optique d'un produit ayant réagi résultant en présence du tensioactif, dans laquelle le tensioactif est un ester d'acide gras et de sorbitan polyoxyéthyléné ou un tribenzylphényléther polyoxyéthyléné, dans laquelle la concentration du tensioactif dans le système de la réaction et/ou de la mesure est de 0,01 à 0,3 %, et dans laquelle l'immunodosage est un dosage d'immuno-agglutination.

4. Utilisation d'un réactif d'immunodosage selon la revendication 3, dans laquelle le réactif comprend en outre un réactif d'immuno-agglutination.

5. Utilisation d'un réactif d'immunodosage selon la revendication 4, dans laquelle le réactif d'immunoagglutination est un réactif d'agglutination de latex.
